(19) Europäisches Patentamt

European Patent Office

Office européen des brevets

(11) **EP 1 097 992 A2**

(12) **EUROPEAN PATENT APPLICATION**

(43) Date of publication:
**09.05.2001 Bulletin 2001/19**

(21) Application number: **00203862.8**

(22) Date of filing: **23.07.1990**

(51) Int Cl.[7]: **C12N 15/11**, C07K 14/435,
G01N 33/542, A61K 49/00,
C12Q 1/68

(84) Designated Contracting States:
**AT BE CH DE DK ES FR GB IT LI LU NL SE**

(30) Priority: **22.07.1989 GB 8916806**

(62) Document number(s) of the earlier application(s) in
accordance with Art. 76 EPC:
**90910819.3 / 0 484 369**

(71) Applicant: **University of Wales College of
Medicine
Cardiff CF4 4XN (GB)**

(72) Inventor: **Campbell, Anthony Keith
Penarth, South Glamorgan CF6 2RF (GB)**

(74) Representative: **Newell, William Joseph et al
Wynne-Jones Laine & James,
33 St Mary Street
Cardiff CF10 1AF (GB)**

Remarks:
This application was filed on 03 - 11 - 2000 as a
divisional application to the application mentioned
under INID code 62.

(54) **Modified bioluminescent proteins and their use**

(57) A bioluminescent protein is disclosed which comprises a bioluminescent protein portion as a donor linked to an energy transfer protein portion as an acceptor by a link portion, said link portion comprising an interaction site for a selected analyte, wherein said analyte reacts with said interaction site The bioluminescent protein is reactive in a bioluminescent reaction to produce light, such that the light is produced having a physical property of a first characteristic when the analyte is interacting with said interaction site, and is produced having a second different characteristic of said property when the analyte is not reacting with said interaction site.

FIG. 1

**Description**

This invention relates to bioluminescent proteins, in particular it relates to bioluminescent proteins which have been modified, for example by chemical means or by genetic engineering. Such modified bioluminescent proteins, hereinafter referred to as "rainbow proteins", nay be used in the detection and quantification of cells, microbes such as bacteria, viruses and protozoa, and substances of biological interest such as substrates, metabolites, intra- and extra- cellular signals, enzymes, antigens, antibodies and nucleic acids.

Bioluminescence is the oxidation of an organic molecule, the "luciferin", by oxygen or one of its netabolites, to emit light. The reaction is catalysed by a protein, usually known as a "luciferase", or a "photoprotein" when the luciferin is so tightly or covalently bound to the luciferase that it does not diffuse off into the surrounding fluid.

$$0_2 + \text{luciferin} + \text{luciferase} \rightarrow \text{oxyluciferin} + \text{light}$$

(or $0_2^-$ or $H_2O_2$) (or photoprotein).

Up to three other substances may also be required to be present in order to generate light, or to alter its colour, and they are as follows:-

(a) A cation such as $H^+$, $Ca^{2+}$, $Mg^{2+}$, or a transition metal such as $Cu^+/Cu^{2+}$, $Fe^{2+}/Fe^{3+}$.
(b) A cofactor such as NADH, FMN, or ATP.
(c) A fluor as an energy transfer acceptor.

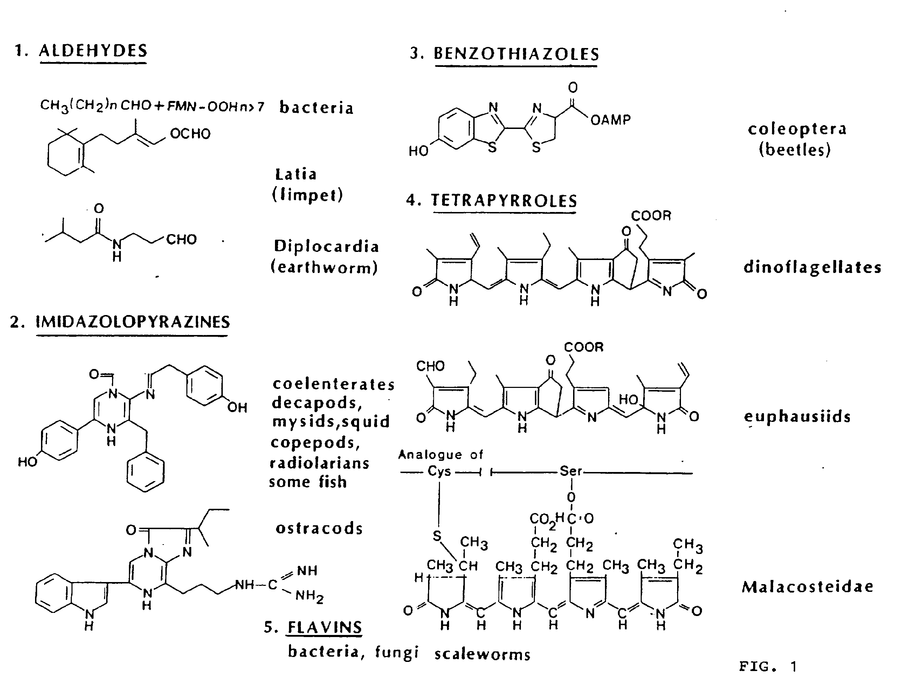

Five chemical families of luciferin have been identified so far (see Figure 1 of the attached drawing):

(a) Aldehydes (found in bacteria, freshwater limpet <u>Latia</u> and earthworms).
(b) Imidazolopyrazines (found in Sarcomastigophora, Cnidaria, Ctenophora, sone Arthropoda, some Mollusca, sone Chordata).
(c) Benzothiazole (found in beetles such as fireflies and glowworms).
(d) Linear tetrapyrroles (found in dinoflagellates, euphasiid shrimp, sone fish).
(e) Flavins (found in bacteria, fungi, polychaete worms and some molluscs).

Reactions involving these luciferins may result in the emission of violet, blue, blue-green, green, yellow or red light and occasionally UV or IR light and such emission may or may not be linearly or circularly polarised. Reference is directed to Chemiluminescence principles and applications in biology and medicine, A.K. Campbell, publ. 1988 Horwood/VCH Chichester Weinheim, for further discussion of bioluminescent reactions.

It has now been found that the light emitted from a bioluminescent reaction involving a modified bioluminescent or "rainbow" protein, may be changed in intensity, colour or polarisation. Such a change can then be used in various assays for detecting, locating and measuring cells, microbes and biological molecules.

In this instance, the cell or substance causes a physical or chemical change,such as phosphorylation, to a rainbow protein such as a genetically engineered luciferase, resulting in a change in intensity, colour or polarisation of the light emission. The bioluminescent reaction is triggered by adding, for example, the luciferin, and modification of the luciferase by the cell or substance being measured causes the reaction to emit light at a shorter or longer wavelength. This enables specific reactions to be detected and quantified in live cells, and within organelles or on the inner or outer surface of the plasma membrane, without the need to break them open, and without the need for separation of bound and unbound fractions.

According to one aspect of the invention there is provided a bioluminescent protein capable of taking part in a bioluminescent reaction to produce light or radiation of altered characteristics under different physical, chemical, biochemical or biological conditions.

The rainbow protein may be produced by the alteration, substitution, addition or removal of one or more amino acids from the end of or within the luciferase or photoprotein. As a result the light emission from the oxyluciferin may be of different colours or different states of polarisation depending on the physical or chemical conditions. A change in colour to another part of the rainbow spectrum may be induced by:

(a) A change in cation concentration such as H , Ca Mg , or transition metal.
(b) A change in anion concentration such as Cl or phosphate.
(c) Covalent modification of the new protein by enzymes causing phospho- or dephosphorylation (including ser/thr, his, and tyr kinases and phosphatases) transglutamination, proteolysis, ADP ribosylation, gly- or glu-cosylation, halogenation, oxidation, methylation and myristilation.

(d) Binding to the rainbow protein of an antigen, an intracellular signal such as cyclic AMP, cyclic GMP, Ip3, Ip4, diacyl glycerol, ATP, ADP, AMP, GTP, any oxy or deoxyribonucloside or nucleotide, a substrate, a drug, a nucleic acid, a gene regulator protein.

(e) Expression of its nucleic acid inside a live cell, as well as its modification or regulation within the cell by gene expression such as promoters, enhancers or oncogenes.

Single or multiple mutations and deletions may be detected in a piece of DNA (eg a PCR product) by linking the "rainbow protein" to one end of the DNA and an energy transfer acceptor or quencher to the other end. Nuclease attack at the mutation will separate the rainbow protein from the acceptor or quencher and thus cause a change in intensity, colour or polarisation of the light emission.

Such alteration, substitution, addition or removal of one or more amino acids may be achieved by chemical means. Alteration of an acid includes alkylation (eg. nethylation), phosphorylation and various other covalent modifications of the type outlined herein. Alternatively the nucleic acid coding for the luciferase or photoprotein may be altered by modifying, substituting, inserting or deleting one or more nucleotides such that the resulting protein has gained or lost a site which interacts with the cations, anions, intracellular signal, covalent modification; proteins or nucleic acid to be measured. The insertion or deletion of nucleotides is normally produced by site directed mutagenesis or by opening up the gene with a specific restriction enzyme, inserting or deleting a selected nucleotide sequence and then sealing up of the gene again or using specific primers with the polymerase chain reaction. The nucleic acid is then transcribed to mRNA and this is then translated to form the rainbow protein either inside a bacterial or eukaryotic cell, or in vitro using, for example, rabbit reticulocyte lysate. The new nucleic acid nay contain an RNA polymerase promoter such as T7, SP6, or mammalian promotors such as actin, myosin, myelin proteins, MMT-V, SV40, antibody, G6P dehydrogenase, and can be amplified in vitro using the polymerase chain reaction. The result is that the rainbow protein can be produced either in a live cell such as a cancer cell, or without cells using enzymatic reactions in vitro. The addition of tissue specific promoter or enhancer sequences to the 5' or 3' end of the DNA coding for the native or altered bioluminescent protein will enable it to be used as a reporter gene and to be expressed specifically in a particular cell or tissue, the expression being detectable by the appearance of a change in light intensity, colour or polarisation.

Another way of producing the DNA for a rainbow protein is to separate into two halves the original DNA for the bioluminescent protein. A piece of DNA or gene is then inserted between the two halves by ligating one half to the 5' end and the other to the 3' end. Alternatively the rainbow protein DIVA could be generated using the polymerase chain reaction so that the sense primer had one part of the rainbow protein DNA linked at 5' end and the antisense primer and the other part linked at the 3' end (i.e. antisense). The pieces of DNA or gene of interest, in the middle, could be from two separate genes. For example one could code for an energy transfer protein, the other for a bioluminescent protein. Only when the two are linked together via a peptide (from DNA/RNA) will the rainbow protein be generated and a shift in colour occur. The energy transfer protein could be any fluor bound covalently or non-covalently to the protein, for example the green fluorescent protein from Aequorea, Obelia, Renilla or other cnidarians, or the blue or yellow fluorescent protein from luminous bacteria, or a flavoprotein, or a phyobiloprotein. The whole protein or just the fluorescent denain may be used. The bioluminescent protein would be any luciferase for example bacterial, firefly, glowworm or copepod, or any photoprotein for example aequorin, obelin or a radiolarin such as thalassicollin.

The protein or its DNA or RNA may be incorporated into a live bacterium or eukaryotic cell by using a virus, plasmid, calcium phosphate transfection, electroporation, liposome fusion or membrane pore forming proteins. Once inside, only live cells with the appropriate biochemistry will produce the "rainbow effect". By incorporating the "rainbow protein" gene into an embryo, oocyte, sperm, seed or seedling a transgenic animal or plant may be produced, enabling gene expression, cell regulation, drug action, or cell damage to be located and measured in individual organs using the "rainbow effect". These new organisms may also be used in hone aquaria, on aeroplane runways, as safe lights at sea, and as house plants.

The rainbow protein may also be incorporated in a different part of the cell by chemical means or genetically engineering the protein to contain a signal peptide which locates it to the inner or outer surface of the plasma membrane or within a particular intracellular organelle (e.g. peroxisone, mitochondrion, chloroplast, endoplasmic reticulum, golgi, secretory vesicle, nucleus or endosome.

Addition of a signal peptide, either chemically or by genetic enginering, will enable the normal or altered luciferase or photoprotein to be targetted into a specific organelle within the cell, or onto the inner or outer surface of the plasma membrane. For example the sequence MLSRLSLRLLSRYLL at the N terminus will locate the bioluminescent protein in the mitochondria, and KKSALLALMYVCPGKADKE on the N terminus will target the protein to the endoplasmic reticulum, a KDEL sequence at the C terminus retaining it there.

The initial luciferase or photoprotein or its gene may come from any of the known chemistries in bioluminescence (see Figure 1) or from the wide range of uncharacterised luminous organisms from more than 700 genera representing at least 16 phyla. The luciferin may be synthesised chemically and added to the biological reaction or cell to generate light. Aternatively, the gene coding for the enzymes responsible for making the luciferin may be linked to the "rainbow protein" gene so that the artificial operon or fusion gene expresses both rainbow protein and makes luciferin in the live cell from nornal cell constituents such as amino acids.

According to a second aspect of the invention there is provided a method of producing a bioluminescent protein by altering, substituting,adding or deleting one or more amino acids to the protein by chemical means or by genetically engineering the nucleic acid coding for the protein.

According to a further aspect of the invention there is provided nucleic acid coding for the bioluminescent protein as hereinbefore defined.

The rainbow protein, or the nucleic acid coding for it, may be used in a range of biological investigations; such as:-

(a) Detection, location and measurement of microbes (protozoa, bacteria, viruses).
(b) Detection and location of cancer cells.
(c) Measurement of enzymes, intracellular signalling and other turnover reactions in cells or fluids.
(d) DNA and RNA binding assays.
(e) Immunoassay and other protein binding assays.

The rainbow proteins and their parent nucleic acids also nay be used in genetic engineering, in the development of transgenic animals, plants and microbes, and in horticulture.

According to yet a further aspect of the invention there is provided the use of a rainbow protein, or the nucleic acid coding for the rainbow protein, for the detection, location or measurement of substances of biological interest such as microbes, cells or biological molecules or reactions therein.

In this aspect, the reaction or substances of biological interest are made to interact with the rainbow protein or its parent nucleic acid. Such interactions include direct or indirect linking such as non-covalent or covalent binding as well as energy transfer processes.

Although the invention has been described above it is to be understood that it includes any inventive combination of the features set out above or in the following description.

The invention nay be performed in various ways and will be further described with reference to the following examples:

EXAMPLE 1

Detection of phosphorylation of a rainbow protein

The peptide leu arg arg ala ser leu gly, known as kemptide, or RTKRSCSVYEPLKT known as malantide was covalently linked to firefly luciferase using disuccinyl suberate at pH8. Addition of 125 ul protein kinase A + cyclic AMP (200 uM) + 125 uM ATP caused phosphorylation of the kemptide, now attached to the luciferase. The resulting shift in colour from yellow-green to red at pH 7.8 or from red to yellow-green at pH 6.5, measured as a ratio in a dual wavelength chemiluminometer, enabled the rate of protein phosphorylation by the kinase to be assayed, and dephosphorylation induced by phosphatase to be assayed subsequently by the reversal of the ratio.

EXAMPLE 2

Engineering Firefly Luciferase

cDNA coding for firefly luciferase was amplified using the polymerase chain reaction (PCR) using 5' sense primers with a T7 RNA polymerase promoter, and 3' antisense primers as follows: Primer code in brackets

```
5' sense primers

(105)   CACCTAATACGACTCACTATAGGGAGAATGGAAGACGCCAAAAAC

(107)   AGAACTGCCTGCCGCAGATTCTCGCA

(110)   ATGCTGTCCCGGCTGTCCCTGCGGCTGCTGTCCCGGTACCTGCTGAAGACGC
        `
        CAAAAAC

(111)   CACCTAATACGACTCACTATAGGGAGAATGCTGTCCCGGCTGTCC
```

**3' antisense primers**

(100)   TCTCGCTGAATACAGTTAC

(106)   CCCCAAGCTTAGATCTCTCTGATTTTTCTTGCGT

(108)   TGCGAGAATCTGCGGCAGGCAGTTCT

The following firefly luciferase cDNA's were constructed using primers in brackets:

(a) full length (105 + 100)
(b) - 36bp i.e. missing peroxisomal signal peptide (105 + 106)
(c) protein kinase A site (RRXS) in middle of protein (step 1: 105 + 108 and 107 + 100; step 2:2 halves from step 1 + 105 + 100)
(d) mitochondrial signal at N terminus (step 1:110 + 100; step 2: step 1 sample + 111 + 100).

The PCR reaction in 50 µl contained 10 mM Tris pH 8.3, 0.01% gelatin, 0.4U Taq polymerase, 2 mM MgCl$_2$, 0.2 mM each dATP, dGTP, dTTP, dCTP, 0.5 µM of each primer, 1 µl DNA (ca 1-100 ng). The reaction, covered with 50 µl mineral oil, was incubated in a Perkin-Elmer thermal cycler for 25 cycles: 94°C 1 minute, 55°C 1 minute, 72°C 2 minutes + 5 seconds extension on each cycle, then for 30 minutes at 37°C with 1U E.coli DNA polymerase (Klenow fragment).

Successful PCR was confirmed by a band on agarose gel electrophoresis. The cDNA was purified by centricon to remove primers, and precipitated in 70% ethanol, 0.7 mM NH$_4$ acetate after extraction with buffered phenol: CHCl$_3$: secondary amyl alcohol (25:24:1). The DNA (0.5-1 µg dissolved in 10mM Tris 0.1 or 1 mM EDTA pH 7.4-7.5) was transcribed in the T7 RNA polymerase in buffer containing 40 mM Tris, pH 7.4-7.5, 6mM MgCl$_2$, 10 mM dithiothreitol, 0.1mg/ml bovine serum albumin, 2 mM spermidine, 0.5 mM each ATP, CTP, UTP, 0.1 mM GTP, 0.5 mM cap m7 G(5') ppp (5') G, 1000 U RNAsin/ml, 800 U T7 RNA polymerase ± 2 µC, $^{32}$P UTP for up to 4 hours (1-2 hours optimal), at 37°C. The reaction was stopped in the ice cold phenol: CHCl$_3$: secondary amyl alcohol (25:24:1), and the RNA precipitated in 70% ethanol + 0.7M NH$_4$Ac, and stored at -20°C.

The RNA was centrifuged, redissolved in 20 µl 70 mM Tris, 1 mM EDTA pH 7.4-7.5 and 1 µl incubated with 5-10 µl rabbit reticulocyte lysate for 1 hour at 30°C to synthesize the luciferase. Luciferase, after dilution, 1/100 is assayed for light emission directly in 50 mM tris, 10 mM MgAc$_2$, 0.1 mg/ml bovine serum albumin, 0.1-0.2 mM luciferin, 0.5-5 mM ATP, pH 7.8, or isolated by isoelectric focusing. The mutant (RRXS) luciferase has a pI of ca 7.1 or 6.8, and the normal luciferase pI ca 6.8. The luciferase with mitochondrial signal also separated from the normal luciferase. On addition of the rabbit reticulocyte lysate containing this altered luciferase it was taken up by added mitochondria, as shown by centrifugation and light emission from the mitochondria and luciferase.

Phosphorylation of the RRXS containing luciferase with protein kinase A, cyclic AMP (0.2 mM), ATP (0.1 - 1 mM) pH7, caused the luciferase to change its pI back towards 6.8, and to shift its colour. The RRXS luciferase had a greener light emission than the native luciferase detected using a dual wavelength chemiluminometer with interference filters (maximum transmission ca 545 and 603 mM).

Primers containing kemptide nucleotide sense or antisense sequence (LRRASLG) or malantide RTKRSGSVYEPLKI were also added either to N or C terminus using a one or two step PCR reaction. These also produced luciferase which could be phosphorylated thereby altering its intensity and colour.

EXAMPLE 3

Preparation of engineered aequorin

cDNA or genomic coding for the Ca$^{2+}$ -activated photoprotein was PCR'd in a similar way to that for firefly luciferase. Using one or two step PCR the protein kinase A recognition peptide kemptide (LRRLALG) or malantide (as Example 2) was added to the N terminus. The mutant aequorin had different kinetic properties enabling protein kinase A to be detected by phosphorylating the altered aequorin (above).

Normal aequorin primers =5' sense TAATACGACTCA CTATAGGGGAGAGAATGGTCAAGCTTTACATCAGACTTCGAC, and 3' antisense GAATTCTTAGGGGACAGCTCCACCGTA. For insertion of kemptide at the N terminus the nucleotide sequence equivalent to LRRASLG was attached to the first 15 bases (including ATG) of the 5' end of aequorin. In step 2, the T7 RNA polymerase promoter was added to form the kemptide-aequorin in vitro for in vitro phosphorylation. Genomic aequorin DNA (made by PCR) was at least as active as that made from mRNA by reverse transcriptase PCR.

EXAMPLE 4

Detection of cancer cells in blood

A blood sample (1 ml) is nixed with a suspension of liposones containing mRNA coding for a rainbow protein catalysing the benzothiazole reaction c in Figure 1. This mRNA was produced as follows:-

The gene coding for firefly luciferase was first isolated from a cDNA library in E. coli using pCDVI plasmid primer + Honjo linker containing SP6 RNA polynerase promoter. A nucleotide sequence GCTCGTCTTATTCAAGATAATGAATATACTGCTCGTTTTGGT representing the phosphorylation site for tyrosine kinase activity of the myc oncogene was inserted at the EcoRI restriction site 30 base pairs downstream from the ATG at the 5' end. The DNA was resealed, recloned and the plasmid insert transcribed by SP6 RNA polymerase in vitro to produce mRNA for the rainbow protein. The original protein produced yellow-green light but the rainbow protein when phosphorylated in the cell produces red light. Thus the presence of cancer cells was detected in blood sample from a leukaemia patient by measuring the ratio of yellow-green (545 nn) to red (603 nm) light in a dual wavelength chemiluminometer.

EXAMPLE 5

Detection of Salmonella

The cDNA for the rainbow protein in Example 4 containing SP6 RNA polymerase promoter was inserted into Salmonella phage. Addition of this phage to Salmonella resulted in expression of the rainbow protein and the generation of red light, enabling as few as 1 bacterium per 20 ml to be detected.

EXAMPLE 6

Detection of HIV RNA

A sample (1 ml) of blood from a patient with AIDS was extracted with 4 M guanidium isothiocyanate and the nucleic acid precipitated with ethanol/NH acetate. An oligonucleotide (10 ul, 1 uM) labelled with a rainbow protein generated from the photoprotein obelin was added to 100 ul redissolved RNA at 50°C and the mixture cooled for 10 minutes at 0°C. The oligonucleotide was specific for a sequence in the HIV coat protein. Binding this to HIV RNA resulted in a shift in the light emission from the rainbow protein from light blue (475 nm) to blue (440 nm). This was detected as a shift in the ratio of light emission at these two wavelengths in a dual photomultiplier chemiluminometer.

EXAMPLE 7

Measurement of testosterone in blood

Testosterone carboxyoxine is reacted with the rainbow protein from the photoprotein obelin to form a testosterone rainbow protein conjugate. 5ul of this containing 1 nmol was incubated with a solution of antibody labelled with fluorescein to testosterone (50ul) pH 7.4 for 30 minutes in the presence or absence of varying concentrations of standard testosterone. The bioluminescent reaction was triggered by addition of Ca and the ratio of light at 475 nm to 530 nn measured. Increasing the concentration of standard testosterone increased the ratio at 475/530. This procedure could be carried out without the need to separate bound from free antigen.

EXAMPLE 8

Detection of Listeria

A sample of suspect food is boiled to extract DNA. A sense primer to the specific Listeria gene or domain covalently coupled to obelin cDNA + 5P6 RNA polymerase pronotor and antisense primer covalently coupled to antisense green fluorescent protein (GFP) cDNA is used to amplify the Listeria gene using the polymerase chain reaction. The result is DNA coding for a new rainbow protein and transcribable by SP6 RNA polymerase.

```
                        Obelin     Listeria     GFP
l----------l--------l---------l----------------l---------l
    promotor          cDNA         gene         cDNA
```

This DNA is transcribed and the mRNA translated using rabbit reticulocyte lysate. Coelenterazine is added to reactivate obelin. The ratio of light at 510/475 nm for rainbow protein versus obelin alone is directly proportional to the amount of Listeria DNA originally present in the food sample. When no Listeria are present the ratio of ratios is 1.

EXAMPLE 9

Measurement of nucleic acid hybridisation by polarisation

The reaction described in Example 6 was carried out but the light emission was detected in a dual photomultiplier chemiluminometer containing two plane polarised filters with the polarisation planes at 90° to each other. The ratio between the two photomultipliers was related to the amount of HIV RNA present.

EXAMPLE 10

Measurement of cyclic AMP or Ip3

Using a two step PCR reaction as described in Examples 2 and 3, the cyclic AMP binding domain from the bacterial CAP protein or the Ip3 binding domain of the endoplasmic reticulum receptor ws added to the N or C terminus or into firefly luciferase or aequorin. The altered proteins were made in vitro fron the PCR DNA product as described in Examples 2 and 3, and characterised by activity and colour of light emission cyclic AMP or Ip3. A change in both intensity and colour enabled CAMP or Ip3 to be measured in cell extracts ot in living cells. Using an image intensifier a CAMP or Ip3 "cloud" could be visualised in this one cell. Similarly the aequorin or luciferase could be seen within the ER or a mitochondrion if it was first made with an ER or mitochondrial signal attached to it (±KDEL) at the C terminus.

It will be appreciated that the bioluminescent protein may be synthesised from amino acid sequences or using a DNA or RNA synthesis techniques, instead of by modification of a protein produced by an organism.

The invention also provides a bioluminescent protein capable of taking part in a bioluminescent reaction to produce light or radiation of altered characteristics under different physical, chemical, biochemical or biological conditions. The altered characteristics may include one or more of colour, polarization state and intensity. The protein may be produced by modification of a bioluminescent protein precursor, for example by the alteration, substitution, addition or removal of one or more amino acids at the N or C terminus or within the protein, and/or by chemical means.

The bioluminescent protein may be responsive to binding or association of the protein with a substance of interest to produce light or radiation of altered characteristics when said bioluminescent reaction is initiated.

The bioluminescent protein may be responsive to covalent or other modification of the protein to produce light or radiation of altered characteristics when said bioluminescent reaction is initiated. Further, the bioluminescent protein may be responsive to the kinetic products of the cellular reaction, to produce light or radiation of altered characteristics when said bioluminescent reaction is initiated.

The bioluminescent protein may include a signal peptide or binding site to target the protein to a particular site within or outside the cell.

The bioluminescent protein may be modified by genetic engineering and made in vitro by in vitro transcription/translation or made in bacteria or archeabacteria, cyanobacteria, blue-green algae, protozoa, fungi, yeast, invertebrate or mammalian or plant cells following transformation or transfection in a suitable vector such as plasmid, bacteriophage, virus, DNA, genomic DNA or mRNA.

The invention also provides a method of producing a bioluminescent protein as defined above by altering, substituting adding or deleting one or more amino acids to the protein by chemical means or by genetically engineering the nucleic acid coding for the protein or a degenerate version thereof.

The invention further provides nucleic acid coding for the bioluminescent protein as defined above or functionally equivalent degenerates thereof. Thus a DNA sequence may include promoter or enhancer sequences specific to the tissue or substance of interest, whereby the sequence may act as a reporter gene. Further, the nucleic acid may include a sequence coding for one or more substances for producing luciferin or a similar molecule capable of emitting light or radiation.

The invention also provides the use of a bioluminescent protein as defined above for the detection, location, measurement or visualization of substances within or outside microbes, whole tissues, whole organisms, cells or biological molecules.

The invention also extends to a method of detecting mutations or detections in a DNA sequence includes linking a bioluminescent protein as defined above to one end of the sequence and an energy transfer acceptor or quencher to the other end.

**Claims**

1. A bioluminescent protein comprising a bioluminescent protein portion as a donor linked to an energy transfer protein portion as an acceptor by a link portion, said link portion comprising an interaction site for a selected analyte, wherein said analyte reacts with said interaction site, said bioluminescent protein being reactive in a bioluminescent reaction to produce light, such that the light is produced having a physical property of a first characteristic when the analyte is interacting with said interaction site, and is produced having a second different characteristic of said property when the analyte is not reacting with said interaction site.

2. A bioluminescent protein according to Claim 1, wherein said energy transfer protein portion and said bioluminescent protein portion are covalently bound via said linker portion.

3. A bioluminescent protein according to Claim 1, wherein said energy transfer protein portion and said bioluminescent protein portion are non-covalently bound via said linker portion.

4. A bioluminescent protein according to Claim 1, wherein said energy transfer protein portion comprises a green fluorescent protein, or a fluorescent domain thereof.

5. A bioluminescent protein according to Claim 1, wherein said energy transfer protein portion comprises a protein or a fluorescent domain thereof, which fluoresces at blue, yellow or higher wavelengths.

6. A bioluminescent protein according to Claim 1, wherein said linker portion comprises a nucleic acid molecule.

7. A bioluminescent protein according to Claim 1, wherein said linker portion comprises a protein or a fragment thereof.

8. A bioluminescent protein according to Claim 1, wherein said linker portion comprises a peptide.

9. Nucleic acid coding for the bioluminescent protein as claimed in Claim 1.

10. A method of monitoring the interaction between a substrate and a substance to which said substrate is exposed, which comprises:-

   coupling a bioluminescent protein portion, capable of taking part in a bioluminescent reaction, to a first portion of said substrate,
   coupling an energy transfer protein portion to a second portion of the substrate, whereby said bioluminescent protein portion and said energy transfer protein portion are in an energy transfer relationship,
   exposing said substrate to said substance,
   initiating said bioluminescent reaction, and
   monitoring at least one of the amount of light emitted directly by said bioluminescent protein portion and the amount of light emitted by said energy transfer protein portion on energy transfer between said bioluminescent protein portion and said energy transfer protein portion.

11. A method according to Claim 10, wherein said substrate comprises a nucleic acid fragment containing one or more mutations or deletions and said substrate comprises a nuclease responsive to said one or more mutations or deletions to cleave said nucleic acid fragment.

12. A method of monitoring the interaction between a ligand and its specific binding partner, which comprises:-

   providing a bioluminescent protein portion capable of taking part in a bioluminescent reaction, coupled to one of said ligand and said specific binding partner;
   providing an energy transfer protein portion coupled to the other of said ligand and said specific binding partner;
   allowing binding to occur between said ligand and its specific binding partner;

initiating said bioluminescent reaction, and monitoring at least one of the amount of light emitted directly by said bioluminescent protein portion and the amount of light emitted by said energy transfer protein portion on energy transfer between said bioluminescent protein portion and said energy transfer protein portion.

13. A method according to Claim 12, wherein said ligand and said specific binding partner are selected from the group comprising:-

nucleic acid probes
proteins
protein fragments, and
peptides.

14. A method of monitoring or detecting a substance in a sample, which comprises:

exposing said sample to:-
a first reagent comprising a bioluminescent protein portion capable of taking part in a bioluminescent reaction and coupled to a first binding portion capable of binding specifically to a first region of said substance; and
a second reagent comprising an energy transfer protein portion coupled to a second binding portion capable of binding specifically to a second region of said substance;
allowing said binding to occur;
initiating said bioluminescent reaction, and,
monitoring at least one of the amount of light emitted directly by said bioluminescent protein portion and the amount of light emitted by said energy transfer protein portion on energy transfer between said bioluminescent protein portion and said energy transfer protein portion.

15. A method according to Claim 14, wherein said ligand and said specific binding partner are selected from the group comprising:-

nucleic acid probes
proteins
protein fragments, and
peptides.

16. A kit, comprising a first reagent including a bioluminescent protein portion capable of taking part in a bioluminescent reaction and coupled to a ligand, and a second reagent including an energy transfer protein portion coupled to a specific binding partner of said ligand.

17. A kit, for use in monitoring or detecting a substance, which comprises:-

a first reagent including a bioluminescent protein portion capable of taking part in a bioluminescent reaction coupled to a first specific binding portion capable of binding to a first region of said substance, and
a second reagent including an energy transfer protein portion coupled to a second specific binding portion capable of binding to a second region of said substance.

18. A kit for monitoring the interaction between a ligand and its specific partner, comprising:-

nucleic acid coding for a first reagent comprising a bioluminescent protein portion coupled to one of said ligand and its specific partner, and
nucleic acid coding for a second reagent comprising an energy transfer protein portion coupled to the other of said ligand and its specific binding partner.

19. A kit for use in monitoring or detecting a substance, which comprises:-

nucleic acid coding for a first reagent comprising a bioluminescent protein portion coupled to a first specific binding portion capable of binding to a first region of said substance, and
nucleic acid coding for a second reagent comprising and energy transfer protein coupled to a second specific binding portion capable of binding to a second region of said substance.

**20.** A method of monitoring a sample for the presence of a nucleic acid fragment of interest, which comprises:-

introducing into said sample a first nucleic acid amplification primer having coupled thereto nucleic acid coding for a bioluminescent protein, and a second nucleic acid primer having coupled thereto nucleic acid coding for an energy transfer protein or a fluorescent domain thereof;
initiating said nucleic acid amplification process;
expressing the product of said nucleic acid amplification process to obtain one or more bioluminescent proteins;
initiating one or more bioluminescent reactions involving said one or more bioluminescent proteins, and monitoring the characteristics of the light emitted during said bioluminescent reaction.

EP 1 097 992 A2

# 1. ALDEHYDES

$CH_3(CH_2)_n CHO + FMN-OOH$ n>7   bacteria

Latia
(limpet)

Diplocardia
(earthworm)

# 2. IMIDAZOLOPYRAZINES

coelenterates
decapods,
mysids, squid
copepods,
radiolarians
some fish

ostracods

# 3. BENZOTHIAZOLES

coleoptera
(beetles)

# 4. TETRAPYRROLES

dinoflagellates

euphausiids

Analogue of
Cys      Ser

Malacosteidae

# 5. FLAVINS

bacteria, fungi  scaleworms

FIG. 1